Europäisches Patentamt

European Patent Office

Office européen des brevets

(11) Publication number: **0 154 104**
A2

## (12) EUROPEAN PATENT APPLICATION

(21) Application number: 84850389.2

(22) Date of filing: 13.12.84

(51) Int. Cl.⁴: **A 61 M 5/14**

(30) Priority: 14.12.83 SE 8306900

(43) Date of publication of application:
11.09.85 Bulletin 85/37

(84) Designated Contracting States:
AT BE CH DE FR GB IT LI NL SE

(71) Applicant: Mediplast AB
Rasundavägen 60
S-171 52 Solna(SE)

(72) Inventor: Ekholmer, Erik
Hammarbacken 1
S-182 35 Danderyd(SE)

(74) Representative: Roth, Ernst Adolf Michael et al,
GÖTEBORGS PATENTBYRA AB Box 5005
S-402 21 Göteborg(SE)

(54) A compressible tube, expecially for infusion solutions.

(57) A compressible tube for transporting liquids especially infusion solutions, a flow regulator being attachable at a preferred position along said tube, said flow regulator regulates the flow area of the tube by pressure action. The object of the invention is to provide a tube which withstands very strongly compressed deformations without the occurence of cold flow and which directly after the discontinuence of the clamping pressure resumes its shape and which is extrudable without any further processing. These objects have been solved by the fact that the tube (10) consists of thinner (11) and thicker (12) wall portions as seen in cross-section, said thinner wall portions (11) being located at the corner portions of a triangle or polygon as seen in cross-section, the connecting portions of which between the corners being formed by the thicker wall portions (12).

FIG 2

A COMPRESSIBLE TUBE, ESPECIALLY FOR INFUSION SOLUTIONS

The present invention refers to a compressible tube for transport of small amounts of liquid, especially for infusion solutions, a flow regulator being attachable on a preferred position along said tube, said flow regulator regulates the flow area of the tube by pressure action.

Background of the invention

Regulation of the flow area in infusion- or transfusion assembleys is mostly performed by a flow regulator, which is passed on the compressible tube and which by clamping reduces the flow area of the tube. Since infusion solutions generally are administered drop by drop the amount of flow is very small. A drawback of all existing flow regulators of the above mentioned kind is that these are very difficult to regulate at small amounts of liquid. A reason for this is that when compressing the tube the midportion thereof will be compressed more than the side edges, so that a 8-shaped area is obtained. Due to the rigidity of the tube material a further compression of the tube by the clamping means will result in a flattening of the midportion of the tube while along the edges of the tube small longitudinal flow channels remain, the sizes of which are uncontrolled and which can be very difficult to regulate. After such a flattening the ability of the tube material to resume its shape is detoriated and in many cases the degree of compression is so high, that so-called cold flow will occur. The strong deformation of the tube involves, that it will take a considerable time for this to return to a lower degree of compression, which means that the tube long after a flow regulation continues to expand and by that change the amount of flow. This can have catastrophic results in case a certain maximum infusion amount has been set for a patient, and which amount after several minutes is increased to the double or multiplied.

Many solutions have been suggested for overcoming this problem, but up to now none of the suggested measures have proved to be reliable. The idea of most of the suggested solutions is to change the flow regulator, while the tube itself has not been a part of the presentation of the problem.

## The object and most important features of the invention

The object of the present invention is to provide a compressible tube of the kind mentioned above, which withstands very strong compressed deformations and which directly after the discontinuance of the clamping pressure resumes its shape. Another object of the present invention is to provide an extrudable tube, which without any processing after the extrusion is ready to use. A further desire is that the manufacture of the new tube should not be more expensive than for conventional tubes for the same purpose. These objects have been solved by the fact that the tube comprises thinner and thicker wall portions as seen in cross section, said thinner portions being located at corner portions of a triangle or polygon as seen in cross section, the straight connecting portions between the corners making said thicker wall portions.

## Description of the drawings

Figure 1 shows in perspective an end view of the fresh not yet solidified extruded tube according to the invention.

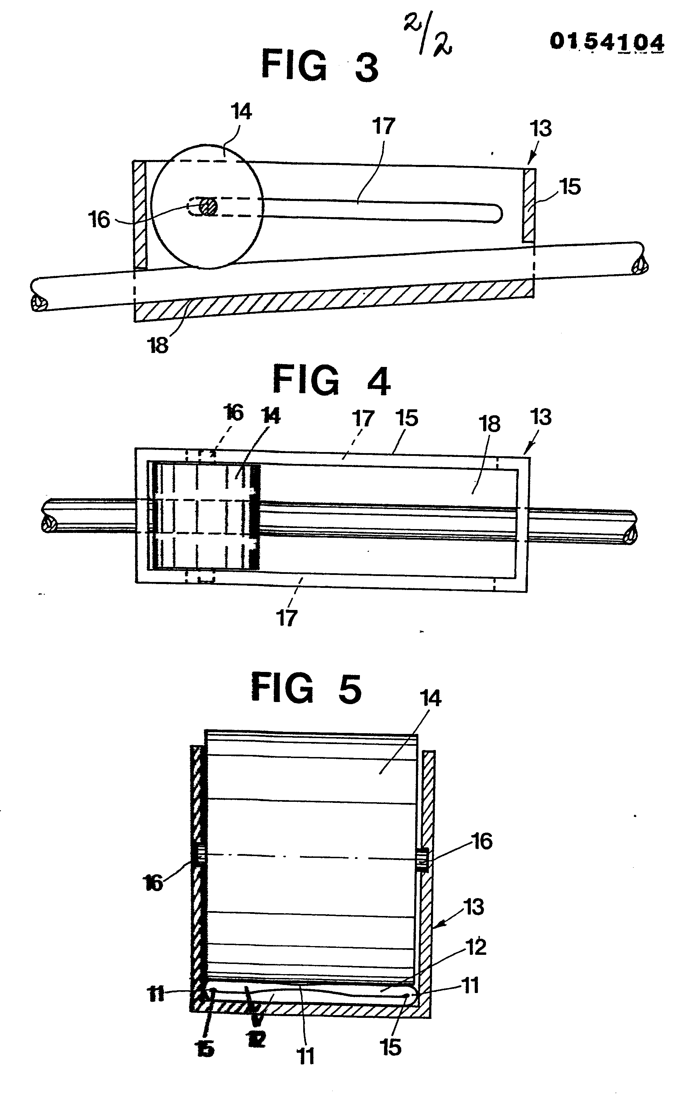

Figure 2 is a view analogous to Figure 1 of the finished, solidified tube according to figure 1.
Figure 3 is a vertical longitudinal section through a known flow regulator.
Figure 4 shows the flow regulator according to figure 3 in a view from above.
Figure 5 shows a section through the flow regulator with a tube according to the invention which is under clamping pressure.

## Description of embodiments

The tube according to the invention, on the drawings denoted
with the numeral 10, is preferably extruded with an outer
circular cross section, while the wall portions of the tube
varies sector by sector. Thus the tube shows in cross section
part-circular, thinner wall portions 11, arranged between
inner, straight, thicker wall portions 12. Due to the inherent
tensions of the material and the variations of thickness of
material a deformation of the material will occur when the
tube is cooled and the plastic material is solidified so far
that the tube assumes a triangular shape in cross section if
it is provided with three part-circular, thinner wall portions
11 and an equal number of straight, thicker portions 12. The
orientation of the thinner portions to the corners of the
geometrical figure occurs during the process of solidification
due to the shrinkage of the material. In case more than three
thin portions are arranged and an equal amount of thick portions, other geometrical figures are obtained, in which always
the thinner portions are located in the corners of the
geometrical figure.

The flow regulator 13, which is of a type commonly used and
comprises a clamp housing 15 and said clamping member 14 dis
placeably arranged therein, which in the shown embodiment consists of a roller. This is on both end sides provided with
axle hubs 16, which are guided in two tracks 17 in the side
walls of the clamp housing 15. The bottom surface 18 of the
clamp housing 15 is inclined with respect to the tracks 17,
against which the bottom surface of the tube 10 rests.

When compressing the tube 10 in the flow regulator 13 by means
of the roller 14, the thinner corner portions 11 will without
diffuculty be bent $180^0$ at the same time as the radius of
curvature of these portions will be very small and by that
also the possible channel 15 formed at the compression. At a
reduction of the pressing force by displacing the roller 14
somewhat to the left according to the drawing, the liquid
pressure within the tube will at first hand act on the thin,
plane portions 11, which are located between the thin, folded
edge portions 11, so that a quick raising of these tube portions is obtained. After that the thicker portions 12 of the side walls will help to raise these so that a practically immediate change of the cross section will occur and not as up to now has been the case, the change of the cross section was continued during 5 minutes or more.

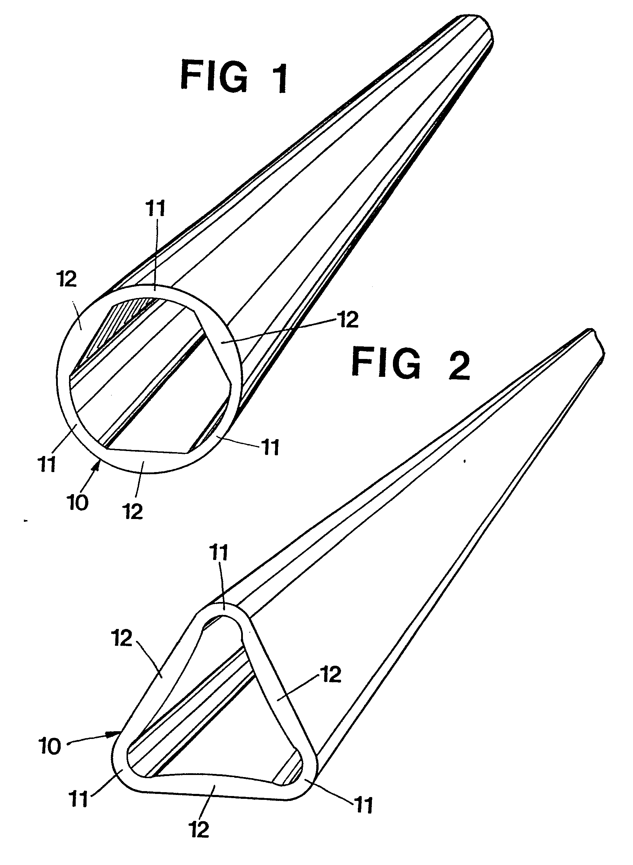

The tube 10 according to the invention has in the fresh extruded state according to figure 1 preferably an outer diameter of about 3,7 mm while the thinner, part circular wall portions 11 have a thickness of about 0,3 mm. The largest thickness of the thicker wall portions 12 is about 0,6 mm.

The invention is not limited to the embodiment shown but a plurality of modifications are possible within the scope of the claims. Thus the tube can have another cross-sectional shape than triangular, for example rectangular, retaining the advantages of the invention.

C L A I M S

1. A compressible tube for transporting small amounts of liquid, especially infusion solutions, a flow regulator being attachable on a preferred position along said tube, said flow regulator regulates the flow area of the tube by pressure action,

c h a r a c t e r i z e d i n,

that the tube (10) comprises thinner (11) and thicker (12) wall portions as seen in cross-section, said thinner portions (11) being located at corner portions of a triangle or polygon as seen in cross-section, the straight connecting portions between the corners being formed by the thicker wall portions (12).

2. A method of manufacturing compressible tubes according to claim 1,

c h a r a c t e r i z e d i n,

that the tube (10) is extruded with an outer or inner circular cross-section and with part-circular, thinner wall portions (11) between straight, thicker wall portions (12), and that the tube extruded in this way is allowed to solidify so that the part-circular thinner wall portions (11) make the corners of a geometrical figure and the straight, thicker portions (12) make its side walls.

# FIG 1

# FIG 2

0154104

# FIG 3

# FIG 4

# FIG 5